Europäisches Patentamt

European Patent Office

Office européen des brevets

(11) Numéro de publication: **0 348 297**

**A1**

## (12) DEMANDE DE BREVET EUROPEEN

(21) Numéro de dépôt: **89401746.6**

(22) Date de dépôt: **20.06.89**

(51) Int. Cl.⁴: **A 61 B 5/02**

(30) Priorité: **21.06.88 FR 8808312**

(43) Date de publication de la demande:
**27.12.89 Bulletin 89/52**

(84) Etats contractants désignés: **CH DE GB IT LI**

(71) Demandeur: **COMMISSARIAT A L'ENERGIE ATOMIQUE**
**31/33, rue de la Fédération**
**F-75015 Paris (FR)**

(72) Inventeur: **Lemaitre, Gérard**
**67 rue Pollet**
**F-93600 Aulnay sous Bois (FR)**

**Schley, Robert**
**10 Bis, Allée de Rond Point**
**F-94310 Orly (FR)**

(74) Mandataire: **Mongrédien, André et al**
**c/o BREVATOME 25, rue de Ponthieu**
**F-75008 Paris (FR)**

(54) **Dispositif de mesure de la tension artérielle.**

(57) Il comprend deux brassards (2, 3) munis chacun d'un manomètre (6, 7) et d'un capteur acoustique (8, 9) dont les mesures sont soustraites pour éliminer les artefacts liés aux mouvements du patient. Le brassard aval (2) est lentement dégonflé lors des mesures et le brassard amont (3) reste à pression constante et faible. Le dispositif présente surtout de l'intérêt pour les mesures sur des patients en activité.

FIG. 1

EP 0 348 297 A1

## Description

### DISPOSITIF DE MESURE DE LA TENSION ARTERIELLE

L'invention a pour objet un dispositif de mesure de la tension artérielle d'un patient en utilisant une mesure corrigée à l'aide des informations de deux capteurs.

De nombreux appareils de mesure de la tension artérielle existent. Le plus connu d'entre eux utilise un brassard gonflable avec un manomètre et une poire et qu'on enfile autour du bras du patient. Le brassard est gonflé à une pression supérieure à la pression systolique puis dégonflé lentement. Quand la pression du brassard a atteint à la pression systolique, le manomètre commence à enregistrer des oscillations de pression ; le médecin détermine ensuite la pression diastolique en continuant à dégonfler le brassard jusqu'à la disparition des oscillations.

Comme cette méthode reste assez empirique, on peut préférer la méthode dite "des bruits de Korotkoff" qui consiste à utiliser également un capteur acoustique qui est plaqué par le brassard contre la peau du patient. Les valeurs des pressions systolique et diastolique sont mesurées à l'aide de modifications des bruits recueillis. Malgré ces avantages, cette méthode n'est encore pas entièrement satisfaisante car elle ne peut s'appliquer qu'à un patient au repos : s'il est en mouvement et à plus forte raison accomplit une quelconque activité manuelle, la tension artérielle est soumise à des irrégularités. Les irrégularités peuvent être engendrées par une variation réelle de la tension artérielle ou par des variations engendrées par un mouvement, que les capteurs enregistrent, dont on peut éventuellement s'apercevoir mais qu'il est fort difficile de corriger exactement.

Avec la présente invention, on offre un perfectionnement sensible aux méthodes de prise de tension artérielle utilisant un brassard gonflable équipé d'un manomètre, ainsi qu'un capteur acoustique, qui offre comme double avantage d'être utilisable par un patient en mouvement et en activité, donc permet d'enregistrer les variations de tension artérielle en cours de journée, tout en fournissant des résultats beaucoup plus précis.

L'idée maîtresse de l'invention consiste à soustraire aux valeurs recueillies par le manomètre des valeurs recueillies par un autre manomètre associé à un autre brassard gonflé à une pression fixe de manière à éliminer les artefacts des signaux dus aux mouvements du patient. Eventuellement, on peut procéder de même pour les indications du capteur acoustique à l'aide d'un second capteur acoustique fixé à l'autre brassard.

Sous sa forme la plus générale, l'invention consiste en un dispositif de mesure de la tension artérielle d'un patient comprenant un premier brassard gonflable que l'on passe autour du bras du patient et qui est équipé d'un premier manomètre et d'un premier capteur acoustique, une source de gaz, un premier circuit pneumatique reliant le premier brassard tour à tour à la source de gaz et à un orifice de dégonflage à débit calibré, caractérisé en ce qu'il comprend un second brassard équipé d'un second manomètre, un second circuit pneumatique pour soit relier le second brassard à la source de gaz, soit relier le second brassard à un orifice de dépressurisation, soit isoler le second brassard, et un circuit électronique recueillant des signaux provenant des deux manomètres et du premier capteur acoustique et soustrayant le signal du second manomètre du signal du premier manomètre.

La source de gaz est généralement une bouteille de gaz comprimé munie d'un détendeur. Le gonflage des deux brassards peut avantageusement être simultané jusqu'à la pression du second brassard. Par ailleurs, les deux brassards peuvent avoir un orifice de dépressurisation commun.

On va maintenant décrire l'invention à l'aide des figures suivantes annexées à titre illustratif et non limitatif :

- la figure 1 est la représentation d'un mode de réalisation préféré de l'invention ; et
- la figure 2 est un diagramme montrant les étapes du fonctionnement de l'invention.

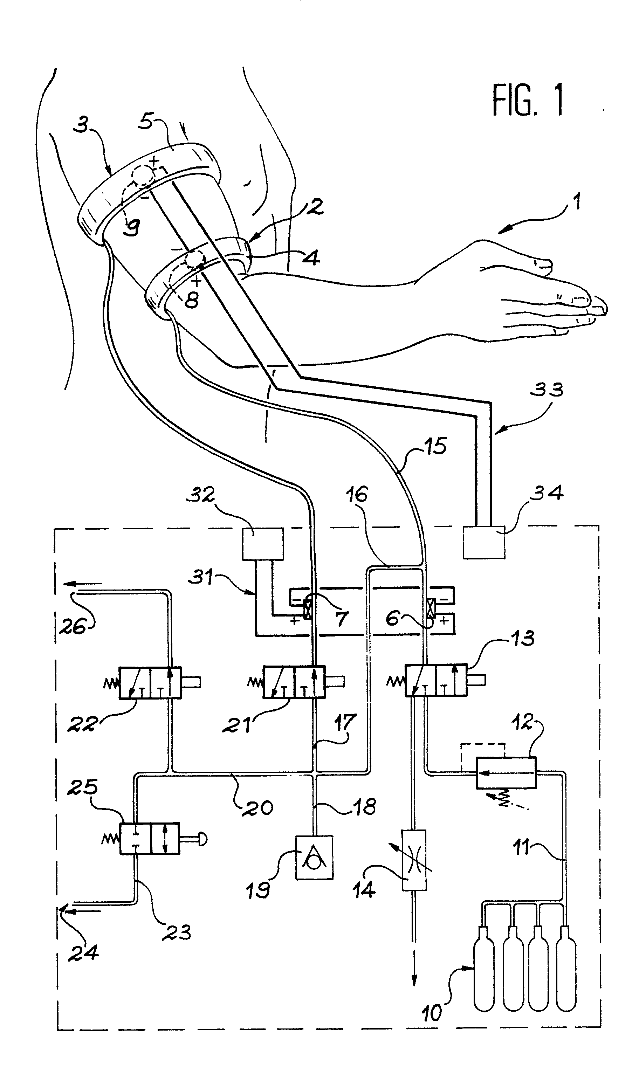

D'après la figure 1, on dispose autour du bras 1 du patient deux brassards : un brassard aval 2 et un brassard amont 3. Les brassards 2 et 3 sont des brassards gonflables du commerce et comprennent chacun une enveloppe élastique, respectivement 4 et 5. Deux manomètres 6 et 7, installés sur les circuits pneumatiques de gonflage que l'on va décrire en détail plus loin, mesurent la pression régnant dans l'enveloppe respective 4 ou 5. Un capteur acoustique, respectivement 8 et 9, vient s'intercaler entre chaque enveloppe 4 et 5 et le bras 1.

Les brassards 2 et 3 sont gonflés à l'aide du gaz contenu dans des bouteilles de Fréon 10 dont le contenu sort par une conduite d'alimentation 11 sur laquelle est situé un détendeur 12 qui détend le Fréon jusqu'à une pression de 300 mbars environ. Un électrodistributeur de gonflage 13 est disposé sur la conduite d'alimentation 11 en aval du détendeur 12 et peut prendre deux positions : une position de gonflage pour laquelle la conduite d'alimentation 11 est ouverte et une position de dégonflage pour laquelle l'écoulement du Fréon hors des bouteilles 10 est interrompu. En position de dégonflage, le Fréon qui se trouve en aval de l'électrodistributeur de gonflage 13 s'écoule librement vers un orifice de dégonflage 14 qui comprend essentiellement un régulateur de débit à pointeau de façon à modifier le débit de dégonflage. Cet écoulement est interrompu quand l'électrodistributeur de gonflage 13 est en position de gonflage.

La conduite de gonflage 11 bifurque en aval de l'électrodistributeur de gonflage 13 : une branche de brassard aval 15 aboutit à l'enveloppe aval 4 et permet son gonflage, alors qu'une autre branche 16 se divise ensuite en une conduite de brassard amont 17 qui aboutit à l'enveloppe amont 5 et permet son gonflage ; une autre dérivation de la branche 16 aboutit à une conduite de soupape 18 aboutissant à une soupape 19 qui empêche une pressurisation trop importante du circuit pneumatique et une conduite de dépressurisation 20.

La conduite de brassard amont 17 comprend un électrodistributeur de pressurisation amont 21 qui peut prendre deux positions : une pour laquelle le Fréon peut librement circuler vers l'enveloppe amont 5 et ainsi, à partir de celui-ci, faire varier la pression dans l'enveloppe amont 5, et une position pour laquelle cette circulation est interrompue et la pression stabilisée.

La conduite de dépressurisation 20, dérivée de la conduite 16, aboutit à un orifice de dépressurisation 26 après avoir passé par un électrodistributeur de dépressurisation 22 qui peut autoriser ou interrompre la circulation de Fréon vers ledit orifice 26.

On peut avantageusement prévoir que la conduite de dépressurisation 20 se divise en amont de l'électrodistributeur de dépressurisation 22 et comporte une branche parallèle 23 qui aboutit à un autre orifice de dépressurisation 24 et sur laquelle une vanne de purge manuelle 25 est placée pour permettre le cas échéant une dépressurisation rapide.

Dans une réalisation concrète, on préconise un détendeur 12 fabriqué par Kunke (référence 48 225 070) et des électrodistributeurs 13, 21 et 22 fabriqués par Clippard Minimatic (références NFEV3M6 et NDEV3M6VDC) ainsi qu'un régulateur de débit 14 de ce même fabricant (référence MNV1).

Les capteurs associés aux brassards 2 et 3 sont montés en série et en opposition de façon à soustraire les signaux qu'ils produisent. Plus précisément, les manomètres 6 et 7 sont reliés par un circuit de mesure de pression 31 qui aboutit à un lecteur de pression 32 et les capteurs acoustiques 8 et 9 sont reliés par un circuit de mesure de bruit 33 qui aboutit à un lecteur de bruit 34.

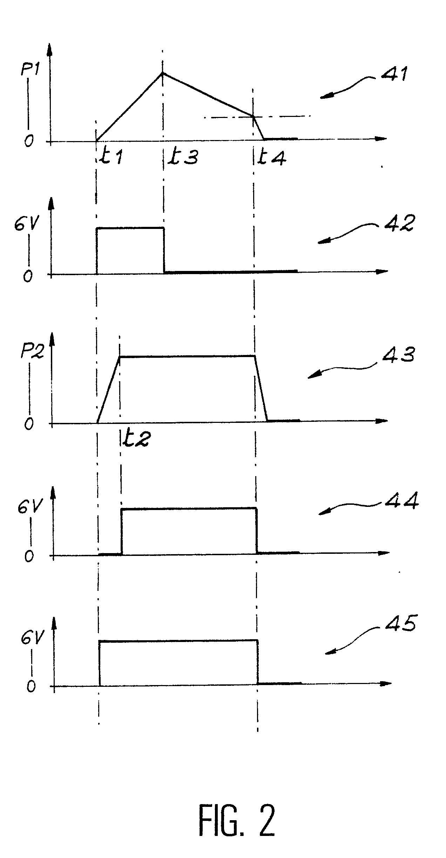

Le fonctionnement de cet appareil va maintenant être décrit à l'aide également de la figure 2 qui représente cinq diagrammes temporels 41 à 45 donnant respectivement, en fonction du temps, la pression dans l'enveloppe amont 5, le cycle d'utilisation de l'électrodistributeur de gonflage 13, la pression dans l'enveloppe aval 4, le cycle d'utilisation de l'électrodistributeur de pressurisation amont 21 et le cycle d'utilisation de l'électrodistributeur de dépressurisation 22.

L'utilisation du dispositif commence à un temps nommé t1 alors que la pression est nulle dans les enveloppes 4 et 5. A ce moment précis, l'électrodistributeur de pressurisation 13 et l'électrodistributeur de dépressurisation 22 sont mis sous tension de façon à ce que le Fréon s'écoule des bouteilles 10 vers les deux brassards 2 et 3 sans pouvoir aboutir aux orifices de dépressurisation 24 et 26. La pression dans les enveloppes 4 et 5 s'élève donc continuellement jusqu'à ce que le manomètre 7 dans le brassard amont 5 détecte qu'une pression prédéterminée est atteinte, pression relativement faible, quelques dizaines de millibars. Un signal est alors envoyé vers l'électrodistributeur de pressurisation amont 21 qui est mis sous tension pour interrompre une pressurisation supplémentaire de l'enveloppe amont 5. On est alors au temps t2.

La pressurisation de l'enveloppe aval 4 se poursuit jusqu'à ce qu'une pression supérieure à la pression systolique, par exemple 300 mbars, soit atteinte au temps t3, et l'électrodistributeur de gonflage 13 est alors mis hors tension pour interrompre la pressurisation de l'enveloppe aval 4 et provoquer au contraire une dépressurisation lente de celui-ci par le régulateur 14. Le lecteur de pression 32 enregistre une diminution régulière de la pression, et ceci même si le patient est en mouvement, car les signaux parasites qui se produisent alors dans les courbes enregistrées par les manomètres 6 et 7 ont exactement la même allure et disparaissent donc par la soustraction. Il en est de même pour les bruits parasites relevés par les deux capteurs acoustiques 8 et 9.

Les mesures sont enregistrées par les circuits de lecture 32 et 34 et mises en mémoire. Elles sont interrompues à un temps t4, où l'électrodistributeur de dépressurisation 22 et l'électrodistributeur de pressurisation amont 21 sont mis hors tension : le Fréon présent dans l'enveloppe amont 5 ainsi que celui encore présent dans l'enveloppe aval 4 s'écoule par l'orifice de dépressurisation 21. On retrouve alors l'état des électrodistributeurs représenté figure 1 et une nouvelle mesure pourra recommencer plus tard.

Ce dispositif présente donc l'avantage de pouvoir être utilisé sur un patient au cours de son activité normale, alors que la tension artérielle au repos n'est pas forcément représentative, et d'enregistrer les variations de tension artérielle en cours de journée : l'ensemble peut être construit suffisamment léger pour pouvoir être porté sans inconvénients.

Les mesures peuvent être déclenchées à tout moment, automatiquement ou à volonté ; les résultats des mesures sont maintenus en mémoire jusqu'au dépouillement périodique par un médecin.

La soupape 19 empêche toute pressurisation excessive due à un déréglage de l'ensemble. En cas de besoin, une dépressurisation plus rapide peut être obtenue par le distributeur manuel 25.

Les quatre petites bouteilles de Fréon 10 peuvent permettre au moins une centaine de mesures avant d'être remplacées ou rechargées.

Le brassard de référence doit de préférence être implanté autour du bras du patient en amont du brassard de mesure.

En effet, un brassard de référence implanté en aval du brassard de mesure risquerait de ne pas permettre de mesurer, à l'aide du manomètre associé à ce brassard de référence, les variations de pression engendrées par le pouls du patient lors de la pressurisation et la dépressurisation, les variations de pression étant stoppées par la pressurisation du brassard de mesure.

**Revendications**

1. Dispositif de mesure de la tension artérielle d'un patient comprenant un premier brassard gonflable (2) que l'on passe autour du bras du patient et qui est équipé d'un premier manomètre (6) et d'un premier capteur acoustique (8), une source de gaz (10), un premier circuit pneumatique reliant le premier brassard (2) tour à tour à la source de gaz (10) et à un orifice de dégonflage à débit calibré (14), caractérisé en ce qu'il comprend un second brassard (3) équipé d'un second manomètre (7), un second circuit pneumatique pour soit relier le second brassard (3) à la source de gaz (10), soit relier le second brassard (3) à un orifice de dépressurisation (26), soit isoler le second brassard (3), et un circuit électronique (31, 32) recueillant des signaux provenant des deux manomètres (6, 7) et du premier capteur acoustique (8), soustrayant le signal du second manomètre (7) du signal du premier manomètre (6).

2. Dispositif de mesure de la tension artérielle selon la revendication 1, caractérisé en ce que la source de gaz comprend une bouteille de gaz comprimé munie d'un détendeur (12).

3. Dispositif de mesure de la tension artérielle selon l'une quelconque des revendications 1 ou 2, caractérisé en ce que les circuits pneumatiques des deux brassards se rejoignent en une partie commune aboutissant à la source de gaz et munie d'un distributeur (13) pour autoriser ou interrompre la circulation du gaz vers les deux brassards (2, 3), le second circuit pneumatique étant en outre muni, hors de la partie commune aux deux circuits, d'un distributeur (21) pour autoriser ou interrompre la circulation du gaz.

4. Dispositif de mesure de la tension artérielle selon l'une quelconque des revendications 1 à 3, caractérisé en ce que le premier circuit pneumatique comprend un orifice de dépressurisation (26) et un distributeur (22) pour autoriser ou interrompre la liaison du premier brassard (2) avec l'orifice de dépressurisation.

5. Dispositif de mesure de la tension artérielle selon la revendication 4, caractérisé en ce que l'orifice de dépressurisation (26) est commun aux deux circuits pneumatiques et le distributeur (22) autorise ou interrompt la liaison avec l'orifice de dépressurisation (26) pour les deux brassards (2, 3) simultanément.

6. Dispositif de mesure de la tension artérielle selon l'une quelconque des revendications 1 à 5, caractérisé en ce qu'il comprend un second capteur acoustique (9) équipant le second brassard et un circuit électronique (33, 34) soustrayant le signal du second capteur acoustique (9) au signal du premier capteur acoustique.

7. Dispositif de mesure de la tension artérielle selon l'une quelconque des revendications 1 à 6, caractérisé en ce que le second brassard (3) est installé en amont du premier brassard (2).

FIG. 1

FIG. 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 146 777  (A.S.J. LEE) <br> * En entier * | 1,4,6 | A 61 B   5/02 |
| A | | 2 | |
| | --- | | |
| Y | US-A-3 348 534  (T.I. MARX et al.) <br> * Colonne 3, ligne 30 - colonne 5, ligne 6; colonne 7, ligne 73 - colonne 8, ligne 6; colonne 8, lignes 38-59; colonne 9, lignes 8-34; colonne 9, ligne 69 - colonne 10, ligne 10; figure 1 * | 1,4,6 | |
| A | | 5 | |
| | --- | | |
| A | US-A-4 649 928  (G.M. SAMARAS et al.) <br> * Colonne 4, lignes 35-57; colonne 5, lignes 3-20; figure 1 * | 1,3,4,6 ,7 | |
| | --- | | |
| A | FR-A-2 562 414  (J.P. LEFEBVRE) <br> * Page 4, ligne 32 - page 5, ligne 36; figure 2 * | 1,3-5 | |
| | --- | | **DOMAINES TECHNIQUES RECHERCHES (Int. Cl.4)** |
| A | DE-A-1 811 401   (PHELPS DODGE COPPER PRODUCTS CORP.) <br> * Page 8, lignes 10-24; page 9, ligne 17 - page 10, ligne 25; page 12, ligne 24 - page 13, ligne 16; figure 1 * | 1-5 | A 61 B |
| | ----- | | |

Le présent rapport a été établi pour toutes les revendications

| Lieu de la recherche | Date d'achèvement de la recherche | Examinateur |
|---|---|---|
| LA HAYE | 22-09-1989 | FERRIGNO, A. |

EPO FORM 1503 03.82 (P0402)